# EUROPEAN PATENT SPECIFICATION

(11) **EP 0 964 704 B1**
(45) Date of publication and mention of the grant of the patent: **20.02.2002**
(21) Application number: 96930482.3
(22) Date of filing: 06.09.1996
(51) Int. Cl.: A61K 48/00, C12Q 1/68

(54) **COMPOSITIONS FOR NUCLEIC ACID TARGETING**
ZUSAMMENSETZUNGEN FÜR NUKLEINSÄURE ZIELRICHTUNG
COMPOSITIONS POUR LE CIBLAGE D'ACIDES NUCLEIQUES

(30) Priority: 08.09.1995 SE 9503117
(43) Date of publication of application: 22.12.1999
(73) Proprietor: Landegren, Ulf, 756 46 Uppsala (SE)
(72) Inventor: Landegren, Ulf, 756 46 Uppsala (SE)
(74) Representative: Bergvall-Eftring, Stina Lena
(86) International application number: SE9601119
(87) International publication number: WO9709069

(56) References cited:
- SCIENCE, Volume 265, Sept. 1994, MATS NILSSON et al., "Padlock Probes: Circularizing Oligonucleotides for Localized DNA Detection", page 2085.

## Description

### Field of the invention

The present invention relates to pharmaceutical compositions for targeting nucleic acid sequences, more specifically double stranded nucleic acid sequences. The compositions comprise oligonucleotides in the form of so called padlock probes. The padlock probes have two free nucleic acid end parts which are at least partially complementary to and capable of hybridizing with two at least substantially neighboring respective regions of a target nucleic acid sequence. Furthermore, the invention relates to use of said compositions as medicaments for treating genetic disorders.

### Background of the invention

Oligonucleotides as potential therapeutics has developed by the ability to synthesize oligonucleotides, chemically modified oligonucleotide analogs and conjugated oligonucleotides, of suitable quantity and purity, as a result of the now ready availability of oligonucleotides through automated synthesis using, for example, the phosphoramidite method.

A first approach to therapeutic use of oligonucleotides is to use them as inhibitors of translation, with the complementary or 'antisense' base sequence targeted to a specific 'sense' sequence in the mRNA. In this way, expression of a specific protein can be regulated or inhibited.

Mechanisms of antisense inhibition include interference with ribosome binding and processing of mRNA conformation or mRNA splicing, and RNAase-H activation of mRNA digestion. The preferred target for antisense inhibition is the 5'-initiation codon.

A second approach to therapeutic use of oligonucleotides is to target DNA therewith and thereby directly inhibit gene function by inhibiting transcription to mRNA. In contrast to mRNA which, although extensively folded, is readily accessible, the DNA duplex is very stable which complicates inhibition thereof.

One way of solving the problem with inaccessibility of double stranded DNA is to take advantage of the fact that a third strand can be accommodated in the major groove of the B-form DNA duplex to form a triplex structure.

Duplex recognition by an oligonucleotide involves the formation of two hydrogen bonds with the purines of Watson-Crick base pairs within the major groove of the double helix. Thymine, cytosine, and guanine can adopt two different orientations called 'Hoogsteen' and 'reverse Hoogsteen' by analogy with the hydrogen-bonding scheme discovered by Hoogsteen in co-crystals of A and T derivatives. In contrast, adenine and inosine can form two hydrogen bonds with and A.T base pair in a single orientation. It should be noted that in order to form two hydrogen bonds with G, cytosine must be protonated. Therefore, triplets involving C+ x G.C are more stable at acidic pH. Methylation at C-5 of cytosine also contributes to stabilization of the triple helix.

Several mechanisms exist by which triple helix formation can alter gene transcription:
1. Triple helix formation within the promoter region can change DNA conformation and therefore alter the rate and efficiency of RNA polymerase initiation. This can lead to either activation or inhibition of transcription.
2. Oligonucleotide binding to a DNA sequence overlapping a transcription factor binding site may inhibit its transactivating capacity.
3. Triplex formation within or adjacent to the region where RNA polymerase binds may inhibit transcription initiation even if RNA polymerase and transcription factors are still bound to the promoter.
4. Oligonucleotide binding downstream of the RNA polymerase recognition site might inhibit progression of the transcription machinery along the DNA and therefore block RNA elongation.

Targeting by triple helix formation is limited to only a particular subset of DNA sequences, such as those associated with homopurine-homopyrimidine tracts.

An alternative way of directly inhibiting DNA is described in Nucleic Acids Research, 1993, Vol 21, No 2, p 197-200 to Nielsen et al. The authors describe that PNA (peptide nucleic acids chimera), i.e., DNA analogues in which the deoxyribose phosphate backbone has been replaced with a peptide backbone consisting of (2-amoniethyl)glycine units have retained the hybridization properties of DNA. There is shown that PNA binds more strongly to complementary oligonucleotides than DNA itself. Moreover, PNA can bind sequence specifically to double stranded DNA. This binding takes place by strand displacement rather than by triple helix formation. In brief, a rather unstable strand displacement complex is first formed with only one PNA molecule bound to the target by Watson-Crick hydrogen bonding, and this is subsequently trapped by binding of a second PNA molecule via Hoogsteen hydrogen bonding.

However, because of their relatively strong binding the sequence specificity rapidly diminishes with the increasing length of the PNA probes.

Branch capture reactions (BCRs) target duplex restriction fragments terminating in overhanging bases with short homologous single stranded DNA oligonucleotides that can pair with the unpaired overhanging bases and some flanking sequence so that complete base pairing displaces the end of one resident strand by branch migration. The limitation of BCRs is that they are limited to targeting only known terminal sequences and are, thus, not very suitable as therapeutic agents.

In Nature Genetics, vol. 3, april 1993, there is described another probe-targeting method which uses Rec A protein-coated short single stranded DNA probes to form four stranded hybrids between probes and duplex DNA targets. With this method internally localized sites can be targeted and the four stranded hybrids are stable.

All the above nucleic acid targeting methods suffer from drawbacks the most important one being the insufficient sequence specificity of the probes. This is an especially essential consideration in respect of the potential use of the probes as therapeutics.

### Summary of the invention

The present invention is derived from the copending international application no. PCT/SE95/00163 entitled: Method, reagent and kit for detection of specific nucleotide sequences. In this application so called padlock probes are described.

In summary, said application describes a probe designed to be circularized in the presence of a target sequence, wherein said probe is caused to close around the target nucleic acid, for example DNA or RNA, such that the cyclic probe will interlock with and thereby be efficiently linked to the target nucleic acid in a manner similar to "padlocks". The circularization of the probe ends is achieved with, for example, ligase. Such covalent catenation of probe molecules to target sequences result in the formation of an extremely stable hybrid.

It has now been surprisingly found that these padlock probes are able to affect gene function directly by binding to double stranded nucleic acids, without a prior denaturation step, and thereby affect the replication and transcription of the bound molecule. This is expected to provide new therapeutic possibilities for in vivo manipulation of gene sequences and treatment of genetic disorders.

The present invention describes a method for targeting double stranded nucleic acids, comprising the following steps:
a) contacting a linear padlock probe having two free nucleic acid end parts which are at least partially complementary to and capable of hybridizing with two at least substantially neighboring respective regions of a target nucleic acid sequence;
   with a double stranded nucleic acid target without prior denaturation of said target;
b) hybridizing said free nucleic acid end parts with said two at least substantially neighboring respective regions of a target nucleic acid sequence; and
c) circularization of said padlock probe by joining said free end parts.

The joining in step c) is performed with a linking agent such as a ligase enzyme or mutually chemically reactive compounds at the free end parts.

According to the present invention provides a pharmaceutical composition for targeting double stranded nucleic acids, comprising an effective amount of a padlock probe oligonucleotide having two free nucleic acid end parts which are at least partially complementary to and capable of hybridizing with two at least substantially neighboring respective regions of a target nucleic acid sequence so that the padlock probe can be circularized by joining said free end parts and catenate with the target sequence for direct inhibition thereof.

The composition is preferably formulated in admixture with a suitable carrier, such as conventional pharmaceutically acceptable carriers known in the art.

According to a second aspect of the invention the above described compositions are used as a medicament for treating genetic disorders.

### Detailed description of the invention

Padlock probe targeting to double stranded DNA according to the invention optionally involves a linking agent which can be chemical or biological. It is, for example, a ligase-assisted reaction. The principle employed in such a reaction is that a linear two-probe segment with a probe in each end, complementary to two target sequences situated in juxtaposition, are joined to a contiguous circular probe sequence with the aid of a linking agent, such as a DNA ligase. Examples of ligases are T4 DNA ligase, T7 DNA ligase, E.coli DNA ligase, and Thermus thermophilus DNA ligase. Also groups that are mutually chemically reactive may be used to join the ends of the probes in an enzyme-independent manner. This way of joining oligonucleotide ends has been previously used in the art. Besides ligases, proteins like RecA or single strand-binding protein can enhance the ability of circularizable probes to hybridize and become catenated to, base paired DNA.

The compositions according to the invention may or may not contain a linking agent depending on the use of the compositions. In vivo, RecA and DNA ligase are already present, and thus the addition of a linking agent may not be necessary for therapeutic applications.

According to the present invention, padlock probes are used in in vitro methods to specifically detect DNA sequences within a cell, without a requirement for prior denaturation. In this manner, for example, the correct spatial relations between specific DNA sequences can be analyzed without artificially induced effects.

In the in vitro approach of the invention, probes of this type could also be used to modify and thereby mutate specific genes in in vitro cell lines, and for instance in embryonal stem cells to give rise to non-human transgenic animals carrying mutations in predefined genes.

In all these various applications, the effects of the padlock probes may be accentuated by at least partially building the probes of non-natural nucleic acids, or of polymers such as PNA, having advantages such as stronger base pairing, greater resistance to nucleases, or increased ability to cross cell membranes.

Padlock probes bind selectively and stably to double stranded DNA and enable sequence specific modification of DNA. In fact, it is contemplated that padlock probes even will be able to selectively bind gene sequence variants with point mutations, in order to inhibit the expression of the mutant genes, since the ligation is dependent upon the exact target sequence. The increased specificity is achieved by the fact that two shorter probe segments have to cooperate for binding to occur. A further advantage is that padlock probes are not sensitive to exonucleases due to their circular shape when they are ligated. On the other hand, excess of padlock probes is rapidly degraded by exonucleases which is a benefit in, for example, drug formulation.

The invention will now be illustrated further, by way of example only, by the following non-limiting specific Examples.

### EXAMPLE 1

### Padlock probe binding to double stranded nucleic acid target

A padlock probe oligonucleotide having the following sequence: 5' P-TGG TGT TTC CTA TGA-((HEG₂)C-B)₄(HEG)₂-AAG AAA TAT CAT CTT-3', wherein P is a phosphate residue, HEG is hexaethylene glycol and C-B is a biotinylated C residue, was synthesized using a commercial DNA synthesizer. The two ends of the oligonucleotide were capable of base-pairing adjacent to each other with exon 9 of the CTFR gene contained in the double stranded plasmid pUC 19.

The probe was labeled by exchanging the present 5' phosphate residue with ³²P using polynucleotide kinase and was allowed to hybridize with the target sequence. In a volume of 20µl 2 pmole probe were mixed with 0.2 pmole of plasmid in the presence or absence of 24 pmole RecA protein in a solution of 10mM Tris, pH 7.5, 10 mM Mg(Ac)₂, 50 mM KAc, 2 mM ATP with 5 units T4 DNA ligase and was incubated for 30 minutes at 37°C.

After incubation, washing was performed under non-hybridizing conditions. Thereafter, the reaction products were separated on a denaturing 6% polyacrylamide gel and the radioactive label was quantified in a Phosphorimager (Molecular Dynamics). The results clearly showed comigration, demonstrating invasion and binding of the above padlock probe to the double stranded plasmid, both in the presence and absence of RecA.

### EXAMPLE 2

### Padlock probe binding to double stranded nucleic acid target and inhibition of promotor

A 90-mer padlock probe with two 20 nucleotide end regions, capable of hybridizing in juxtaposition on one strand of the insert cloned in a Bluescript plasmid, was allowed to hybridize to a denatured, amplified fragment of the insert, and including the two transcriptional promoters T3 and T7, flanking the insert. One ng of amplification product was mixed with 20 pmol of padlock probe in a 10µl reaction with 10U of *Tth* ligase (Epicenter Technologies) in the presence of a NAD+-containing buffer, as recommended by the manufacturer. This buffer was previously shown to be well suited also for transcription by both the T3 and T7 RNA polymerases. The presence of a padlock probe on the double stranded amplified fragment efficiently interferred with transcription of both strands of the amplified fragment.

## Claims

1. A pharmaceutical composition for targeting double stranded nucleic acids, **characterized in that** it comprises an effective amount of a padlock probe oligonucleotide having two free nucleic acid end parts which are at least partially complementary to and capable of hybridizing with two at least substantially neighboring respective regions of a target nucleic acid sequence so that the padlock probe can be circularized by joining said free end parts and catenate with the target sequence for direct inhibition thereof.

2. A composition according to claim 1, in admixture with a suitable carrier.

3. A composition according to claim 1, also comprising a linking agent.

4. A composition according to claim 3, **wherein** linking agent is a ligase enzyme.

5. A composition according to claim 1, comprising mutually chemically reactive compounds at said end parts.

6. A composition according to any of claims 1-5, **wherein** said padlock probe comprises non-natural nucleic acids or polymers.

7. A composition for targeting nucleic acids, comprising an effective amount of a padlock probe having two free nucleic acid end parts which are at least partially complementary to and capable of hybridizing with two at least substantially neighboring respective regions of a target nucleic acid sequence so that it can be circularized and catenate with the target sequence, for use as a medicament.

## Patentansprüche

1. Pharmazeutische Zusammensetzung zur zielgerichteten Erkennung von doppelsträngigen Nukleinsäuren, **dadurch gekennzeichnet, dass** sie eine wirkame Menge einer Oligonukleotidsonde nach Art eines Vorhängeschlosses mit zwei freien Nukleinsäureendteilen enthält, die mindestens teilweise zu zwei mindestens im Wesentlichen benachbarten jeweiligen Bereiche einer Ziel-Nukleinsäuresequenz komplementär sind und mit diesen hydridisieren können, so dass die Vorhängeschloss-Sonde zirkularisiert werden kann, indem die freien Endteile mit der Zielsequenz für deren direkte Inhibierung verbunden und verkettet werden.

2. Zusammensetzung nach Anspruch 1 im Gemisch mit einem geeigneten Träger.

3. Zusammensetzung nach Anspruch 1, die ebenfalls ein Verknüpfungsmittel enthält.

4. Zusammensetzung nach Anspruch 3, worin das Verknüpfungsmittel ein Ligaseenzym ist.

5. Zusammensetzung nach Anspruch 1, die an ihren Endteilen Verbindungen aufweist, die gegenseitig chemisch reaktiv sind.

6. Zusammensetzung nach einem der Ansprüche 1 bis 5, worin die Vorhängeschloss-Sonde nichtnatürliche Nukleinsäuren oder Polymere enthält.

7. Zusammensetzung zur zielgerichteten Erkennung von Nukleinsäuren, die eine wirksame Menge einer Sonde nach Art eines Vorhängeschlosses mit zwei freien Nukleinsäureendteilen enthält, die mindestens teilweise zu zwei mindestens im Wesentlichen benachbarten jeweiligen Bereichen einer Ziel-Nukleinsäuresequenz komplementär sind und mit diesen hybridisieren können, so dass sie mit der Zielsequenz zirkularisiert und verknüpft werden kann, für die Verwendung als Medikament.

## Revendications

1. Composition pharmaceutique pour cibler les acides nucléiques doubles brins, **caractérisée en ce qu'**elle comprend une quantité efficace d'une sonde d'oligonucléotide en forme de cadenas (« padlock probe oligonucleotide ») présentant deux parties d'extrémités d'acides nucléiques libres qui sont au moins partiellement complémentaires et capables d'hybrider au moins deux régions respectives sensiblement voisines d'une séquence d'acides nucléiques ciblé telle que la sonde en forme de cadenas peut circuler en joignant lesdites parties d'extrémités et en s'enchaînant avec la séquence cible pour une inhibition directe de celle-ci.

2. Composition pharmaceutique selon la revendication 1, **caractérisée en ce qu**'elle est mélangée avec un support approprié.

3. Composition pharmaceutique selon la revendication 1, **caractérisée en ce qu**'elle comprend aussi un agent de liaison.

4. Composition pharmaceutique selon la revendication 3, **caractérisée en ce que** l'agent de liaison est une enzyme synthétase.

5. Composition pharmaceutique selon la revendication 1, **caractérisée en ce qu**'elle comprend des composés chimiques mutuellement réactifs audites parties d'extrémités.

6. Composition pharmaceutique selon l'une quelconque des revendications 1-5, **caractérisée en ce que** ladite sonde en forme de cadenas comprend des polymères ou acides nucléiques non-naturels.

7. Composition pour cibler des acides nucléiques, **caractérisée en ce qu'elle** comprend une quantité efficace d'une sonde en forme de cadenas présentant deux parties d'extrémités d'acides nucléiques libres qui sont au moins partiellement complémentaires et capables d'hybrider au moins deux régions respectives sensiblement voisines d'une séquence d'acides nucléiques cible tel qu'elle peut circuler et être enchaînée avec la séquence cible, pour une utilisation comme médicament.
